# EUROPÄISCHE PATENTSCHRIFT

(11) **EP 0 724 643 B1**
(45) Veröffentlichungstag und Bekanntmachung des Hinweises auf die Patenterteilung: **30.06.2004**
(21) Anmeldenummer: 94928873.2
(22) Anmeldetag: 07.10.1994
(51) Int. Cl.: C12N 15/87, C12N 7/04, C12N 15/62

(54) **ENDOSOMOLYTISCH WIRKSAME PARTIKEL**
ENDOSOMOLYTICALLY ACTIVE PARTICLES
PARTICULES A ACTIVITE ENDOSOMOLYTIQUE

(30) Priorität: 14.10.1993 DE 4335025
(43) Veröffentlichungstag der Anmeldung: 07.08.1996
(73) Patentinhaber: Boehringer Ingelheim International GmbH, 55218 Ingelheim am Rhein (DE)
(72) Erfinder: COTTEN, Matthew, A-1130 Wien (AT); CHIOCCA, Susanna, A-1030 Wien (AT); SCHAFFNER, Gotthold, A-1040 Wien (AU); WAGNER, Ernst, A-2103 Langenzersdorf (AT)
(86) Internationale Anmeldenummer: PCT/EP1994/003313
(87) Internationale Veröffentlichungsnummer: WO 1995/010624

(56) Entgegenhaltungen:
- EP-A- 0 545 016
- WO-A-88/03562
- WO-A-93/07282
- WO-A-94/00588
- WO-A-94/06923
- THE JOURNAL OF GENERAL VIROLOGY, Bd.74, Nr.4, April 1993, READING, UK Seiten 541 - 548 MASTICO ET AL. 'Multiple presentation of foreign peptides on the surface of an RNA-free sperical bacteriophage capsid' in der Anmeldung erwähnt

## Beschreibung

Die Erfindung bezieht sich auf das Einbringen von Nukleinsäuren in höhere eukaryotische Zellen.

In den letzten Jahren hat der therapeutische Ansatz der Gentherapie für die Behandlung zahlreicher Erkrankungen an Interesse gewonnen. Die Gentherapie wird eingesetzt, um in vivo therapeutisch wirksame Genprodukte zu synthetisieren, wodurch z.B. im Falle eines genetischen Defekts die Funktion des fehlenden Gens ersetzt wird. Beispiele für genetisch bedingte Erkrankungen, bei denen die Gentherapie einen erfolgversprechenden Ansatz darstellt, sind Hämophilie, beta-Thalassämie und "Severe Combined Immune Deficiency" (SCID), ein Syndrom, das durch einen genetisch bedingten Mangel des Enzyms Adenosindeaminase hervorgerufen wird. Anwendungsmöglichkeiten bestehen ferner bei der Immunregulation, mittels einer Impfung eine humorale oder intrazelluläre Immunität erzielt wird. Weitere Beispiele für genetische Defekte, bei denen eine Verabreichung von Nukleinsäure, die für das defekte Gen kodiert, z.B. in individuell auf den Bedarf abgestimmter Form verabreicht werden kann, sind Muskeldystrophie (Dystrophin-Gen), Cystische Fibrose ("Cystic fibrosis transmembrane conductance regulator gene"), Hypercholesterolämie (LDL-Rezeptor-Gen). Gentherapeutische Behandlungsmethoden können auch eingesetzt werden, wenn Hormone, Wachstumsfaktoren oder cytotoxisch oder immunmodulierend wirkende Proteine im Organismus synthetisiert werden sollen.

Die Gentherapie stellt ferner einen erfolgversprechenden Ansatz für die Behandlung von Krebs dar, wobei sog. "Krebsvakzine" verabreicht werden. Um die Immunogenizität von Tumorzellen zu erhöhen, werden diese verändert, um sie entweder stärker antigenisch zu machen, oder um sie zu veranlassen, bestimmte immunmodulierende Substanzen zu erzeugen, z.B. Zytokine, die dann eine Immunantwort auslösen. Um dies zu bewirken, werden die Zellen mit DNA transfiziert, die für ein Zytokin, z.B. IL-2, IL-4, IFN-gamma, TNF-α, kodiert. Die am weitesten fortgeschrittenen Techniken für den Gentransfer in autologe Tumorzellen benützen virale Vektoren.

Nukleinsäuren als therapeutisch wirksame Substanzen kommen außerdem zur Anwendung, um bestimmte Zellfunktionen zu inhibieren, z.B. haben sich Antisense RNAs und -DNAs oder Ribozyme als wirksame Mittel für die selektive Inhibierung bestimmter Gensequenzen erwiesen.

In jüngerer Zeit wurden Gentransfersysteme entwickelt, die die Beschränkungen der retroviralen und adenoviralen Vektoren umgehen und deren Sicherheitsrisken, die aufgrund des Co-Transfers von lebensfähigen viralen Genelementen des Ursprungsvirus bestehen, ausschalten. Diese Gentransfersysteme beruhen auf Mechanismen, deren sich die Zelle für den Transport von Makromolekülen bedient, z.B. auf dem äußerst leistungsfähigen Weg der Rezeptor-vermittelten Endozytose (Wu und Wu, 1987; EP-Al 0 388 758;
WO 91/17773, WO 92/17210 und WO 92/19281). Mit Hilfe dieser Methode, die sich bifunktioneller molekularer Konjugate bedient, die eine DNA-Bindungsdomäne und eine Domäne mit Spezifität für einen Zelloberflächenrezeptor aufweisen, konnten hohe Gentransferraten erzielt werden.

Da der Gentransfer auf physiologischem Weg, wie ihn die Rezeptor-vermittelte Endozytose mittels Nukleinsäure-Komplexen darstellt, große Vorteile aufweist (nichttoxischer Mechanismus des Durchtritts durch die Zellmembran; Möglichkeit der Verabreichung biologisch aktiver Nukleinsäuren, auf repetitiver oder kontinuierlicher Basis; Möglichkeit des zellspezifischen Targeting; Herstellbarkeit der Konjugate in großen Mengen), besteht das Bedürfnis, dieses System effizienter zu machen.

Bei der Anwendung der Gentransfertechniken, die auf dem Prinzip der Rezeptor-vermittelten Endozytose beruhen, stellte sich heraus, daß ein limitierender Faktor des Systems der Abbau des Genmaterials in der Zelle nach dessen Freisetzung aus den Endosomen ist. Eine wesentliche Verbesserung des Systems wurde daher durch eine Technik erzielt, die die Fähigkeit von bestimmten Viren und Viruskomponenten ausnützt, Endosomen aufbrechen zu können. Mit Hilfe eines Zusatzes dieser endosomolytischen Mittel konnte eine erhebliche Steigerung der Expressionsraten der in die Zelle importierten Gene erzielt werden (Wagner et al., 1991a und 1991b; Cotten et al., 1992; Wagner et al., 1992a und 1992b; Zatloukal et al., 1992; Cotten et al., 1993a und 1993b; Curiel et al. 1991; WO 93/07283 und WO 93/07282).

Als endosomolytische Agentien wurden neben Viren oder Viruskomponenten u.a. synthetische Peptide vorgeschlagen, die von viralen, pH-abhängigen, membranaktiven Peptiden, wie z.B. dem Influenza A Hämagglutinin-Fusionspeptid, abgeleitet sind. Synthetische Transfektionskomplexe, enthaltend entweder das Influenzapeptid (WO 93/07283, Wagner et al., 1992) oder verschiedene Peptide auf der Grundlage des GALA-Peptids (Subbarao et al., 1987; Parente et al., 1990; und WO 93/07283) zeigten die Brauchbarkeit dieser Peptide.

Die Anwendung synthetischer membranaktiver Peptide als endosomolytische Mittel ist jedoch begrenzt. Dies könnte daran liegen, daß sie in den wahllos angeordneten chemisch oder ionisch gebundenen Gentransferkomplexen möglicherweise nicht immer in einer Form zugänglich vorliegen, die die Ausübung ihrer Funktion ermöglicht.

Den zufällig angeordneten Konjugaten aus synthetischen Peptiden und Polylysin, die für die Komplexierung und Kondensierung der DNA-Moleküle verwendet wurden, fehlt eine geordnete dreidimensionale Strukturierung der endosomolytischen Funktion, wie sie das hinsichtlich seiner endosomolytischen Aktivität äußerst effiziente Adenoviruspartikels aufweist. Von der endosomolytischen Aktivität des Adenoviruspartikels wird angenommen, daß sie in der Pentonbasis lokalisiert ist (Seth et al., 1984), welche in einer definierten Kopienzahl an spezifischen Stellen auf der Oberfläche des Viruspartikels vorliegt (Stewart et al., 1993). Diese organisierte Anordnung könnte eine Funktion im Hinblick auf die Erfordernisse für den Zusammenbau eines Viruspartikels haben, sie könnte aber ebenso eine Rolle bei der Steuerung der Wechselwirkungen des membranaktiven Motivs auf den Pentonproteinen mit der Endosomenmembran spielen.

Der vorliegenden Erfindung lag die Aufgabe zugrunde, endosomolytische Mittel bereitzustellen, die eine Verbesserung der Gentransfersysteme über Rezeptor-vermittelte Endozytose ermöglichen, indem sie hohe Expressionsraten bei weitestgehender. Ausschaltung von Sicherheitsrisken gewährleisten.

Bei der Lösung der gestellten Aufgabe wurde von der Überlegung ausgegangen, Verbesserungen der endosomolytischen Eigenschaften der aus dem Stand der Technik bekannten membranaktiven Peptide dadurch zu erzielen, daß man sie in einer geordneten Form zusammenbaut oder anordnet, indem man sie auf einem Protein anbringt, das die Fähigkeit hat, sich selbst zu Teilchen mit geordneten Strukturen zusammenzubauen.

Im Zuge der Realisierung der vorliegenden Erfindung wurden zunächst die leeren Kapside getestet, wie sie in normalen Adenovirusinfektionen gefunden werden (Daniell, 1976). Es zeigte sich, daß leere Adenoviruskapside keine endosomolytische Fähigkeit aufweisen; diese dürfte erst durch die proteolytische Prozessierung, die in einer späten Phase der Virusreifung stattfindet, aktiviert werden (Weber, 1976). Diese Annahme ist im Einklang damit, daß ein Adenovirusstamm, der hinsichtlich der Prozessierung temperaturempfindlich ist (Ad2 ts1; Weber, 1976) bei der restriktiven Temperatur unreife Teilchen produziert, die nicht fähig sind, die Freisetzung von co-endozytiertem Material auszulösen (Defer et al., 1990). In Vorversuchen zur vorliegenden Erfindung wurde außerdem festgestellt, daß diese Partikel nicht die Fähigkeit haben, den DNA-Transport zu verbessern oder Liposomen aufzubrechen. Falls dieses bei Adenoviren beobachtete Merkmal ein allgemeines Charakteristikum der Virusreifung ist, ist zu erwarten, daß leere Kapside nicht die endosomolytische Aktivität der ganzen reifen Viruspartikel aufweisen.

Die vorliegende Erfindung betrifft ein endosomolytisch wirksames virusähnliches Partikel, das aus Einheiten von Kapsidproteinen, abgeleitet von Kapsidproteinen von Viren oder virusähnlichen Teilchen, zusammengesetzt ist, wobei die Kapsidproteinheiten mit einer membranaktiven peptidischen Sequenz modifiziert sind.

Das Gerüst der erfindungsgemäßen Partikel ist ein leeres Viruskapsid oder ein kapsidartiges Partikel aus Proteinen von Viren oder virusähnlichen Teilchen, wie Bakteriophagen oder Hefe-Transposons. Die Proteine, die das Kapsidgerüst bilden, werden im Rahmen der vorliegenden Erfindung als "Kapsidproteine" bezeichnet.

Die membranaktive Peptidsequenz ist auf den Kapsidproteineinheiten so angeordnet, daß gewährleistet ist, daß sie am Ort ihrer Wirksamkeit, also in der Zelle, funktionell verfügbar ist: die membranaktiven peptidischen Sequenzen liegen entweder an der Oberfläche der Partikel frei oder sind in einer Weise in der Oberflächenstruktur angeordnet, daß ihre Freilegung durch Ereignisse in der Zelle, wie Proteolyse, pH-Wertänderung oder Änderung des Redoxpotentials, freigelegt wird. Die membranaktive Funktion der Peptide, die u.a. durch ihre Zugänglichkeit bestimmt wird, äußert sich in ihrer endosomolytischen Aktivität. Diese schlägt sich in der Steigerung der Gentransferkapazität nieder und kann mittels Gentransferexperimenten getestet werden.

Leere Kapside von einfachen Viren ohne Hülle sind von einer Reihe von Viren verfügbar. Die natürlichen Kapside bestehen im allgemeinen aus ein bis drei Proteinen. Die Herstellbarkeit der Kapsidproteine in großen Mengen, z.B. im Baculovirussystem, und deren Fähigkeit zum Selbstzusammenbau haben die Gewinnung von virusähnlichen Partikeln mit geordneten Strukturen, ähnlich denen des nativen Virus, ermöglicht. In den meisten Fällen sind die Partikel frei von Nukleinsäure. (Leere Kapside, die von sich aus endosomolytische Aktivität aufweisen, die die Aufnahme und die Expression von in die Zelle transportierter DNA verbessert, können als solche ohne weitere Modifikation des Partikels verwendet werden, wie in der WO 93/07283 vorgeschlagen wurde. Ein Beispiel dafür sind die leeren Kapside des Parvovirus B19, die z.B. durch Baculovirusexpression erhalten werden können.)

Die erfindungsgemäßen Partikel sind von Kapsidstrukturen abgeleitet, die von sich aus die zur Steigerung der Effizienz des Gentransfers erforderliche endosomolytische Aktivität nicht oder nicht in ausreichendem oder gewünschtem Ausmaß aufweisen.

Die das Gerüst bildenden Ausgangspartikel können natürlichen Ursprungs sein, in diesem Fall werden sie insbesondere von Virusinfektionen erhalten.

Vorzugsweise werden die Partikel auf rekombinantem Weg hergestellt, indem die, gegebenenfalls modifizierten, Kapsidproteine exprimiert und gereinigt und, falls sie nicht bereits in assoziierter Form vorliegen, anschließend assoziieren gelassen werden.

Wenn nicht-modifizierte Kapsidproteine exprimiert werden, können die nach ihrer Assoziation erhaltenen Partikel, die das Gerüst bilden, nachträglich an ihrer Oberfläche mit den membranaktiven Peptiden modifiziert werden, z.B. auf chemischem Weg durch Kopplung mit synthetischen membranaktiven Peptiden. Die Kopplung des membranaktiven Peptids an das Kapsidgerüst kann in für die Kopplung von Peptiden an sich bekannter Weise erfolgen, z.B. auf chemischem Weg, wobei, falls erforderlich, die Einzelkomponenten vor der Kopplungsreaktion mit Linkersubstanzen versehen werden. Die Kopplung kann z.B. erfolgen über Disulfidbrücken, die unter reduzierenden Bedingungen wieder gespalten werden können (z.B. bei Kopplung mittels Succinimidylpyridyldithiopropionat; Jung et al., 1981).

Falls das Kapsid geeignete Kohlenhydratketten aufweist, kann es mit dem Peptid über diese Kohlenhydratketten verbunden werden. Dabei kann nach der für die Herstellung von Glykoprotein-Polykation-Konjugaten in der WO 92/15281 beschriebenen Methode vorgegangen werden.

Ein weiteres Verfahren zur Herstellung der erfindungsgemäßen Partikel ist die enzymatische Kopplung des membranaktiven Peptids an das Gerüstkapsid durch eine Transglutaminase. Dabei kann nach der in der WO 93/07283 für die Kopplung von Polylysin an Adenovirus beschriebenen Methode vorgegangen werden. Voraussetzung hierfür ist, daß an Proteinen entsprechende Glutamine oder Lysine vorhanden sind, die von dem Enzym umgesetzt werden können.

Vorzugsweise werden die erfindungsgemäßen Partikel erhalten, indem die modifizierten Kapsidproteine mit Hilfe rekombinanter Methoden hergestellt werden.

Die Erfindung betrifft somit in einem weiteren Aspekt ein Verfahren zur Herstellung von endosomolytisch wirksamen virusähnlichen Partikeln, wobei man eine für ein Kapsidprotein von Viren oder virusähnlichen Teilchen kodierende DNA, die mit einer für ein membranaktives Peptid kodierenden Sequenz modifiziert ist, exprimiert und das erhaltene Kapsidprotein, falls erforderlich, zu Kapsidstrukturen assoziieren läßt.

Die chimäre DNA, die eine für das Kapsidprotein und eine für das membranaktive Peptid kodierende Sequenz enthält, wird z.B. in mit Baculoviren transformierten Insektenzellen, in Hefe oder in Bakterien, exprimiert. Das erhaltene, durch eine membranaktive Peptiddomäne modifizierte Kapsidprotein kann z.B. assoziieren gelassen werden, nachdem die überexprimierten Kapsidproteinmonomeren denaturiert, gereinigt und das Denaturierungsmittel entfernt wurden. Als Denaturierungsmittel kommen insbesondere Harnstoff oder Guanidinhydrochlorid, gegebenenfalls in Gegenwart milder Detergentien und/oder Reduktionsmittel, in Betracht. Eine Denaturierung ist dann nicht erforderlich, wenn sich die modifizierten Kapsidproteine bereits im Wirtsorganismus zu Kapsidstrukturen anordnen, wie dies offensichtlich beim Hefe-Ty-Partikel der Fall ist. In diesem Fall werden die Wirtszellen mechanisch aufgebrochen und die fertigen Kapsid-Teilchen geerntet.

Bezüglich des Expressionssystems, von dem eine große Auswahl zur routinemäßigen Verwendung zur Verfügung steht, bestehen keine Beschränkungen. Bevorzugt wird ein System eingesetzt, das die Expression der modifizierten Kapsidproteine in großen Mengen ermöglicht; wegen ihrer Effizienz werden im allgemeinen bakterielle Expressionssysteme bevorzugt. Ein Beispiel für ein im Rahmen der vorliegenden Erfindung geeignetes bakterielles Expressionssystem ist das von Studier et al., 1990, beschriebene. Ein Beispiel für ein geeignetes Hefe-Expressionssystem ist das von Emr, 1990, beschriebene; auch Baculovirussysteme, die für die Herstellung von Kapsidproteinen schon verschiedentlich herangezogen wurden, sind geeignet (z.B. O'Reilly et al., 1992). Es können konstitutive oder induzierbare Expressionssysteme verwendet werden. Durch Auswahl und gegebenenfalls Modifikation von vorhandenen Expressionssystemen kann gesteuert werden, in welcher Form die Kapside erhalten werden, z.B. kann eine Verringerung der Expressionsrate die Denaturierung und anschließende Resolubilisierung der Kapsidproteine überflüssig machen.

Die rekombinante Herstellung, bei der die Modifikation der Kapsidproteine im Zuge der Expression der entsprechend modifizierten DNA erfolgt, hat gegenüber der nachträglichen Modifikation von Kapsiden den Vorteil, daß die Lage der membranaktiven Domänen auf dem Protein und das Verhältnis Kapsidproteine zu membranaktiven Peptiden exakt definierbar ist.

Bei der rekombinanten Herstellung der modifizierten Kapsidproteine ist grundsätzlich zu beachten, daß die Gegenwart des membranaktiven Peptids, sei es aufgrund seiner Sequenz oder Anordnung, die Fähigkeit der exprimierten Kapsidproteine, sich zu geordneten Strukturen zusammenzufügen, nicht beeinträchtigt. Diese Forderung gilt auch hinsichtlich der Stelle im Kapsidprotein, in die das Peptid eingefügt ist.

Für den Fall, daß die Kapside aus mehr als einem Protein bestehen, können die Proteine co-exprimiert werden; z.B. im Fall von zwei Kapsidproteinen durch Co-Transformation des Wirtsorganismus mit zwei Plasmiden, die je eine Kapsidprotein-Sequenz tragen, oder durch Transformation des Wirts mit einem doppelt rekombinanten Vektor, der die beiden Sequenzen trägt, wie z.B. für die Expression des Kapsids des Parvovirus B19 in Insektenzellen mittels Baculoviren gezeigt wurde (Brown et al., 1991).

Zu den derzeit verfügbaren, im Rahmen der vorliegenden Erfindung verwendbaren Kapsiden, deren Proteine im Baculovirus-System exprimiert werden und die sich zu Partikeln zusammensetzen können, zählen das Adenoassoziierte Virus, ein Dependovirus (Ruffing et al., 1992); "Aleutian Mink Disease Virus", ein autonomes Parvovirus (Christensen et al., 1993); Flock House Virus, ein Nodovirus (Schneeman et al., 1993), Papilloma Virus; Poliovirus (Urakawa et al., 1989); Norwalk Virus (Jiang et al., 1992) und Polyomavirus. Beispiele für weitere Kapsidproteine, die durch Einführen von Fremddomänen modifiziert werden können, sind das des L-A-Teilchens aus Hefe (Icho und Wickner, 1989; Wickner, 1993), der Bakteriophagen Qβ, GA, SP und anderer Phagen der Familie der Leviviridae, und des Bakteriophagen phi x 174 (Ackerman und DuBow, 1987).

Besonders gute Voraussetzungen bringen das Hefe-Ty-Teilchen und der MS2-Phage mit: Kingsman et al., 1991, haben gezeigt, daß geordnete Hefe-Ty-Partikel für die immunogene, multivalente Darbietung von Peptiden und kleinen Proteinen wie HIV gp120-Epitopen, Hepatitis B-Antigenen, etc. geeignet sind. Dies ist möglich, weil das Hauptprctein des Ty-Teilchens, das Produkt des TyA-Gens, die Fähigkeit besitzt, sich selbst zu 40 nm großen isometrischen Partikeln zusammenzusetzen (Burns et al., 1992). Kingsman et al. haben ein Fragment des Proteins identifiziert, das für diese Selbstzusammenbau-Aktivität ausreicht; Peptidsequenzen, die an den Carboxy-Terminus dieses Proteins angehängt werden, liegen an der Oberfläche des zusammengesetzten Partikels frei. Das MS2-Virus besitzt ein 24 nm quasiicosohedrales Kapsid, bestehend aus 180 Kopien des Haupt-Hüllproteins (13 kd), einer Kopie des Reifungsproteins (45 kd), und ein 3569 nt RNA-Genom. MS2 war der erste Organismus, dessen komplettes Genom sequenziert wurde (Fiers et al., 1976). Die Kristallstruktur dieses Virus wurde aufgeklärt und zeigte eine freiliegende β-Haarnadelschleife auf der Oberfläche jedes der 180 Kapsomeren (Valegard et al., 1990). Die nicht-fixierte Struktur dieser Schleife und deren Freiliegen an der Oberfläche wurde ausgenützt, um Partikel für die Antigenpräsentation zu bilden, indem Abschnitte aus 11 bis 26 Aminosäuren in diese Schleife eingefügt wurden (Mastico et al., 1993). Das MS2-Kapsidprotein toleriert diese Insertionen unter Beibehaltung seiner Fähigkeit, sich zu geordneten Kapsidstrukturen selbst zusammenzusetzen; das Kapsidprotein kann in Harnstoff denaturiert werden und baut sich nach dessen Entfernung zu nativen virusähnlichen Strukturen zusammen (Mastico et al., 1993). Dadurch wird die Zusammensetzung von Kapsiden, die an ihrer Oberfläche zahlreiche Peptidepitope tragen, ermöglicht.

An das Partikel im allgemeinen werden die Anforderungen gestellt, da3 es eine Größe von weniger als 100 nm, vorzugsweise weniger als 50 nm Durchmesser aufweisen soll, um die Aufnahme mittels Endozytose zu ermöglichen, daß es frei ist von überflüssiger, insbesondere infektiöser Nukleinsäure, die keine Funktion für den Gentransfer aufweist, und daß es die Voraussetzung mitbringt, daß ihm eine endosomolytische Aktivität (in Form von membranaktiven Peptiden oder kleinen Proteinen) verliehen werden kann, entweder auf chemischem Weg oder mittels genetischer Manipulation.

An rekombinant herzustellende Kapside im besonderen werden die folgenden Anforderungen gestellt: Das Kapsid soll eine möglichst einfache Struktur, vorzugsweise bestehend aus maximal drei Untereinheiten, aufweisen. Die Untereinheiten sollen sich entweder bereits im Wirtsorganismus, in dem sie exprimiert werden, von selbst assoziieren oder, falls sie nach Überexpression in unlöslicher Form vorliegen, resolubilisierbar sein und sich von selbst zu Kapsidstrukturen zusammensetzen. Der Einfachheit halber wird von Kapsidproteinen ausgegangen, deren Gene in klonierter Form verfügbar sind, andernfalls muß die Klonierung erst vorgenommen werden.
Im Hinblick auf die Insertionsstelle für die Einfügung der membranaktiven Peptidsequenz wird bevorzugt von Kapsidstrukturen ausgegangen, deren 3D-Struktur (Kristallstruktur) mittels Röntgenstrukturanalyse aufgeklärt wurde. An den Sequenzbereich im Kapsidprotein, in dem die Anbringung der membranaktiven Peptidsequenz vorgenommen wird, wird generell die Anforderung gestellt, daß er für die Ausbildung der Kapsidstruktur bzw. den Zusammenbau keine Funktion hat bzw. daß ihre Funktion durch die Einfügung einer Peptidstruktur nicht behindert wird. Wenn die Kristallstruktur verfügbar ist, wird diese bei der Festlegung der Stelle für die Einfügung der Peptiddomäne herangezogen: Zeigt die Kristallstruktur, daß das Virus oder virusähnliche Partikel an seiner Oberfläche Schleifen hat, wie z.B. der MS2-Phage, so werden bevorzugt diese Schleifen, die für die Ausbildung der Kapsidstruktur nicht erforderlich sind, für die Modifikation herangezogen.
Ist die Kristallstruktur nicht bekannt, kann bei der Bestimmung der Insertionsstelle empirisch vorgegangen werden: Aus der Aminosäuresequenz kann abgeleitet werden, ob bestimmte Bereiche gegebenenfalls die Grundlage für die Ausbildung von Strukturelementen, wie α-helikalen oder von β-Faltblattstrukturen, darstellen. Sofern Informationen über Deletionen oder künstliche oder natürliche Mutationen von Virusproteinen einen Rückschluß auf die Existenz nicht-konservierter Regionen zulassen, können die nicht konservierten Regionen, die für die Struktur nicht erforderlich sind, wie z.B. die Schleifen des MS2-Phagen, für die Modifikation benützt werden.

Bei der Auswahl von Kapsidgerüsten und membranaktiven Peptiden sowie der Bestimmung der Insertionsstelle der Peptidsequenz für die Konstruktion der erfindungsgemäßen Partikel wird z.B. wie folgt vorgegangen: Die für das bzw. die Kapsidprotein(e) kodierende(n) DNA-Sequenz(en) werden, unter der Kontrolle geeigneter Expressionskontrollsequenzen, in einen Vektor eingebracht und in einem geeigneten Wirt exprimiert. Parallel dazu werden Vektoren zur Expression gebracht, in denen jeweils die Kapsidproteinsequenz mit einer für ein membranaktives Peptid kodierenden Sequenz modifiziert ist. Dabei wird gegebenenfalls zunächst die Insertionsstelle für die Fremdsequenz variiert, um die optimale Stelle zu ermitteln. Im Zuge der Aufarbeitung der Expressionsprodukte können die Bedingungen für die Reinigung der Kapsidmonomeren und für deren Assoziation optimiert werden.

Im Anschluß daran wird in einer Serie von Transfektionsversuchen, in denen als DNA zweckmäßigerweise ein Reportergen eingesetzt wird, unter ansonsten identischen Transfektionsbedingungen die Fähigkeit der nicht-modifizierten und der mit verschiedenen membranaktiven Peptiden, die gegebenenfalls an verschiedenen Stellen des Kapsidproteins eingefügt wurden, modifizierten Kapside getestet, den Transport der Reporter-DNA in die Zelle zu verbessern.

Grundsätzlich sind sämtliche membranaktive Peptide für die Herstellung der erfindungsgemäßen Partikel geeignet, sofern sie die Bedingung erfüllen, den Zusammenbau der Kapsidstrukturen nicht zu beeinträchtigen und dem Partikel in der Zelle die endosomolytische Funktion zu verleihen, die die Steigerung der Gentransfereffizienz mit sich bringt. Zu geeigneten membranaktiven Peptiden, die entweder nachträglich an die Ausgangspartikel gekoppelt oder deren kodierende DNA-Sequenz zur Herstellung chimärer Kapsidprotein-DNA verwendet werden kann, zählen z.B. die von Subbarao et al., 1987; Parente et al., 1990 und von Wagner et al., 1992, beschriebenen Peptide sowie die natürlichen und synthetischen membranaktiven endosomolytisch wirksamen Peptide, die in der WO 93/07283 beschrieben sind.

Die Eignung der Hefe-Ty-Partikel, Fremdpeptide an der Oberfläche zu präsentieren, wurde im Rahmen der vorliegenden Erfindung ausgenutzt, um, ausgehend von Hefe-Ty-Elementen (Boeke et al., 1988), genetisch modifizierte Partikel zu erhalten, die auf ihrer Oberfläche membranaktive Peptide aufweisen.

Die Erfindung betrifft somit in einem bevorzugten Aspekt ein Hefe-Ty-Partikel, zusammengesetzt aus TyA-Protein-Einheiten, die mit einer membranaktiven Peptidsequenz modifiziert sind.

In einer bevorzugten Ausgestaltung ist das Ty-Partikel mit der Peptidsequenz Glu Ala Ala Leu Ala Glu Ala Leu Ala Glu Ala Leu Ala Glu His Leu Ala Glu Ala Leu Ala Glu Ala Leu Glu Ala Leu Ala Ala (GALA) modifiziert, die sich am Carboxy-Terminus des TyA-Proteins befindet.

In einer weiteren bevorzugten Ausgestaltung ist das Ty-Partikel mit der Peptidsequenz Gly Leu Phe Glu Ala Ile Glu Gly Phe Ile Glu Asn Gly Trp Glu Gly Leu Ala Glu Ala Leu Ala Glu Ala Leu Glu Ala Leu Ala Ala Gly Gly Ser modifiziert, die sich am Carboxy-Terminus des TyA-Proteins befindet.

Die erfindungsgemäßen Hefe-Ty-Partikel werden erhalten, indem man eine DNA, kodierend für das TyA-Protein, das am Carboxy-Terminus eine membranaktive Peptid-Sequenz aufweist, exprimiert, die Wirtszellen aufschließt und die Kapside erntet. Die Expression der modifizierten TyA-Sequenz, z.B. in Hefe oder Bakterien, liefert modifizierte Ty-Partikel, die aufgrund ihrer Größe und Dichte gereinigt werden können.

Die gereinigten endosomolytischen Ty-Partikel wurden im Rahmen der vorliegenden Erfindung biotinyliert und mit Streptavidin-Polylysin und Transferrin-Polylysin sowie der in die Zelle zu transportierenden DNA zu ternären Transfektionskomplexen vereinigt.

Alternativ wurden die endosomolytischen Ty-Partikel mittels Transglutaminase direkt mit Polylysin gekoppelt und mit Transferrin-Polylysin-Konjugaten und der DNA zu ternären Transfektionskomplexen vereinigt.

Ferner wurde ihm Rahmen der vorliegenden Erfindung die Toleranz des MS2-Phagen für die Insertion von Fremdsequenzen in die zwischen den β-Faltblättern gelegene Schleife in der Nähe des N-Terminus des MS2-Kapsidproteins ausgenützt, indem seine Kapsidproteine modifiziert wurden, um membranaktive Peptidsequenzen auf seiner Oberfläche zu exprimieren.

Eine weitere bevorzugte Ausführungsform der vorliegenden Erfindung ist somit ein MS2-Partikel, zusammengesetzt. aus MS2-Kapsidprotein-Einheiten, die mit einer membranaktiven Peptidsequenz modifiziert sind.

Vorzugsweise ist das membranaktive Peptid in der β-Haarnadelschleifenregion zwischen Aminosäure 11 (Asp) und Aminosäure 17 (Asp), insbesondere zwischen Aminosäure 14 (Gly) und 15 (Thr) des MS2-Kapsidproteins eingefügt.

Eine weitere mögliche Insertionsstelle für das membranaktive Peptid liegt in der C-terminalen Region des MS2-Kapsidproteins.

In einer bevorzugten Ausgestaltung der Erfindung ist das endosomolytische MS2-Partikel mit der Peptidsequenz GALA, die zwischen Aminosäure 14 und Aminosäure 15 des MS2-Kapsidproteins eingefügt ist, modifiziert.

Die erfindungsgemäßen MS2-Partikel werden bevorzugt erhalten, indem die durch Einfügung der für das membranaktive Peptid kodierende Sequenz modifizierte Kapsidprotein-DNA exprimiert, und das erhaltene modifizierte Kapsidprotein denaturiert und unter Entfernung des Denaturierungsmittels assoziieren gelassen wird.

Die gereinigten endosomolytischen MS2-Partikel wurden im Rahmen der vorliegenden Erfindung biotinyliert und mit Streptavidin-Polylysin und Transferrin-Polylysin sowie der in die Zelle zu transportierenden DNA zu Komplexen vereinigt.

In einer bevorzugten Ausführungsform weisen die erfindungsgemäßen Partikel zusätzlich zu dem/den membranaktiven Peptid(en) eine Peptidsequenz mit der Funktion eines Liganden für die Zielzelle auf. Damit wird dem erfindungsgemäßen Partikel neben seiner endosomolytischen Funktion eine Internalisierungsfunktion verliehen; diese Peptidsequenz wird im folgenden "Ligandenpeptid" bezeichnet.

Das am besten charakterisierte Ligandenpeptid ist die Arginin-Glycin-Asparaginsäure-Sequenz (RGD), die in verschiedenen Integrin-bindenden Zelladhäsionsproteinen, wie Fibronektin, Fibrinogen, von Willebrand Faktor und Vitronektin (Pierschbacher und Ruoslahti, 1984; 1987), gefunden wurde. Von einem RGD-Motiv, das in der Pentonbasis von Adenovirus Typ 2 und Typ 5 vorhanden ist, wurde gezeigt, daß es bei der Internalisierung des Virus eine Rolle spielt (Wickham et al., 1993). Von einer synthetischen, eine DisulfidBrücke enthaltenden, in ihrer Struktur fixierten Version der RGD-Sequenz, die das Muster Cys-(Xaa)₆-Cys benützt, wobei die sechs Aminosäuren neben der RGD-Sequenz drei andere Aminosäuren entsprechend den von Pierschbacher und Ruoslahti, 1987, und O'Neil et al., 1992, definierten Regeln enthalten, wurde festgestellt, daß sie eine um drei Größenordnungen höhere Affinität für ein Integrinsubstrat aufweist als eine nicht-fixierte Version der Sequenz (O'Neil et al., 1992).
Eine für dieses Motiv kodierende Sequenz wurde auch in das M13-Gen III eingeführt, um es an der Oberfläche des filamentösen Phagen zu präsentieren.

Eine derartige kurze, das RGD-Motiv enthaltende Ligandenpeptidsequenz kann in Kapsidproteine eingefügt werden, um erfindungsgemäße Partikel zu erhalten, die auf ihrer Oberfläche ein zellbindendes Motiv aufweisen.

Die Fähigkeit z.B. des MS2-Kapsidproteins, sich aus Harnstoff-denaturierten Monomeren selbst zusammenzusetzen, kann auch dazu ausgenutzt werden, MS2-Partikel herzustellen, die mehr als eine Fremddomäne aufweisen. Voraussetzung dafür ist auch in diesem Fall, daß diese Insertionen die Fähigkeit der Partikel zum Selbstzusammenbau nicht beeinträchtigen. Um ein erfindungsgemäßes Partikel zu erhalten, das einerseits eine membranaktive Domäne (z.B. das GALA-Motiv) und andererseits eine zellbindende Domäne (z.B. das RGD-Motiv) aufweist, wird vorzugsweise so vorgegangen, daß einerseits Kapsidmonomere mit einer membranaktiven Modifikation und andererseits solche mit einer Ligandenmodifikation hergestellt werden, und die beiden unterschiedlich modifizierten, denaturierten Monomeren in einem definierten Mengenverhältnis gemischt und das Denaturierungsmittel entfernt wird, um die Assoziation der modifizierten Proteine zu virusähnlichen Partikeln zu ermöglichen.

Alternativ zu dem RGD-Motiv können andere Ligandenpeptide in die Kapsidmonomeren eingeführt werden; Beispiele dafür sind kleine Peptidwachstumsfaktoren und Hormone, wie das EGF(Epidermal Growth Factor)-Peptid , Insulin, das costimulatorische Molekül HSA "Heat Stable Antigen" (Kay et al., 1990), ferner Peptide von sog. Superantigenen, kodiert vom Maus-Mammatumor-Virus (Torres et al., 1993).

In einer bevorzugten Ausführungsform sind die erfindungsgemäßen Partikel mit einer Nukleinsäure-bindenden Domäne, insbesondere einer organischen polykationischen Verbindung wie Polylysin, versehen. Als Nukleinsäure-affine Substanzen kommen auch andere organische Polykationen, wie sie z.B. in der WO 93/07283 vorgeschlagen sind, in Betracht.

In dieser Ausführungsform der vorliegenden Erfindung enthalten somit die virusähnlichen Partikel zusätzlich zu den membranaktiven endosomolytischen Peptiden und gegebenenfalls den zellbindenden Liganden-Motiven Domänen, die die Fähigkeit haben, an Nukleinsäuren zu binden.

Diese Partikel, die eine DNA-Bindungsdomäne enthalten, können hergestellt werden, indem das Kapsid nachträglich mit einer DNA bindenden Substanz wie Polylysin konjugiert wird.

Die Konjugation des Kapsids z.B. mit Polylysin kann nach für die Kopplung von Peptiden mit Polyaminverbindungen an sich bekannter Weise erfolgen, z.B. auf chemischem Weg, durch Kopplung über eine Biotin-Streptavidin-Brücke oder durch direkte Bindung des Polylysins an das Kapsid mittels Transglutaminase. Dabei kann analog vorgegangen werden, wie in der WO 93/07283 für die Kopplung von Polylysin an Viren oder Viruskomponenten beschrieben.

Alternativ zur nachträglichen Konjugation von Kapsiden mit einem DNA-bindenden Peptid kann die Modifikation der Kapsidproteine mit einer DNA-Bindungsdomäne auch direkt erfolgen, d.h. durch Expression einer chimären DNA-Sequenz, bestehend aus einer für das Kapsidprotein kodierenden DNA-Sequenz, und einer für das DNA-bindende Peptid kodierenden Sequenz.

Bei dieser Herstellungsmethode wird an die DNA-Bindungspeptide die auch für die anderen Fremddomänen geltende Anforderung gestellt, daß ihre Gegenwart auf dem Kapsidprotein dessen Fähigkeit, sich zu geordneten Strukturen zusammenzusetzen, nicht beeinträchtigt.

Beispiele für DNA-bindende Motive, die nach Expression der chimären Kapsid-DNA auf den erfindungsgemäßen Partikeln vorliegen, sind kationische Polypeptide, z.B. die Homologen Polylysin, Polyarginin, oder Peptide, die von natürlich vorkommenden DNA-bindenden Proteinen, wie Histonen, Core-Proteinen von Adenovirus (z.B. Protein V, Protein VII und das 13 kd Protein L211K) oder Protaminen, abgeleitet sind.

Das Vorliegen einer polykationischen Domäne in Form von Polylysin ermöglicht die Komplexbildung der erfindungsgemäßen Kapsid-Konjugate mit der in die Zelle zu transportierenden Nukleinsäure.

Die Herstellung von erfindungsgemäßen Partikeln, die mehr als eine Fremddomäne aufweisen, z.B. mehrere membranaktive Domänen oder eine membranaktive Domäne in Verbindung mit einer Liganden- und/oder einer DNA-bindenden Domäne, können in zwei oder mehr getrennten, gleichen oder verschiedenen Expressionssystemen hergestellt werden.

So kann z.B. ein Kapsidprotein-Monomeres mit einer membranaktiven Domäne, z.B. dem Peptid GALA, einerseits und ein Kapsid-Monomeres mit einer Ligandendomäne, z.B. dem RGD-Motiv, andererseits hergestellt und die Monomeren im gewünschten Verhältnis gemischt werden, damit diese sich zu geordneten Strukturen zusammenzubauen. Das optimale Mischungsverhältnis wird empirisch bestimmt.

Die erfindungsgemäßen Partikel werden als endosomolytische Mittel in Zusammensetzungen für den Gentransfer eingesetzt, wie sie in der WO 93/07283 beschrieben sind.

Die Erfindung betrifft somit in einem weiteren Aspekt eine Zusammensetzung für den Transport von Nukleinsäure in die höhere eukaryotische Zelle, in denen die Nukleinsäure komplexiert ist mit endosomolytisch wirksamen virusähnlichen Partikeln, bestehend aus modifizierten Einheiten von Kapsidproteinen, abgeleitet von Viren oder virusähnlichen Teilchen, wobei die Kapsidproteineinheiten membranaktive peptidische Sequenzen sowie polykationische Sequenzen zur Bindung der Nukleinsäure aufweisen.

In einer bevorzugten Ausführungsform enthalten die Gentransferkomplexe zusätzlich zu den erfindungsgemäßen endosomolytischen Partikeln, die eine Nukleinsäure-Bindungsdomäne aufweisen, ein Konjugat, in dem eine Nukleinsäure-bindende Domäne, im allgemeinen dieselbe wie die des Partikels, mit einem Internalisierungsfaktor für die zu transfizierende Zielzelle gekoppelt ist. Diese ternären Komplexe oder Kombinationskomplexe werden vor allem dann angewendet, wenn das endosomolytische Partikel nicht von sich aus in die Zielzelle eindringen kann, d.h. wenn es nicht in nativer Form in die Zelle eindringen kann und auch nicht mit einer Ligandendomäne für die Zielzelle modifiziert wurde. Diese Ausführungsform kann jedoch auch zum Einsatz kommen, wenn die Ligandenfunktion eines erfindungsgemäßen Partikels durch eine zusätzliche Ligandenfunktion ergänzt werden soll.

Bevorzugt im Rahmen der vorliegenden Erfindung sind Transfektionskomplexe, bestehend aus DNA, dem erfindungsgemäßen, mit Polylysin konjugiertem Partikel, und einem Trarisferrin-Polylysin-Konjugat.

In den Transfektionskomplexen kann zusätzlich eine Nukleinsäure-bindende Substanz, insbesondere Polylysin, in nicht-konjugierter Form enthalten sein, um die Nukleinsäure zu kondensieren. In diesem Fall hat die in dem erfindungsgemäßen Partikel bzw.

Internalisierungsfaktor-Konjugat enthaltene Nukleinsäure-Bindungsdomäne die Funktion des Anhaftens an die Nukleinsäure, ohne dabei die Gesamtheit der negativen Ladungen abzusättigen.

Bezüglich der Definition des Begriffes "Internalisierungsfaktor" sowie der Anwendbarkeit von Internalisierungsfaktor-Konjugaten zusammen mit den erfindungsgemäßen endosomolytischen Partikeln in ternären Transfektionskomplexen wird auf die Offenbarung der WO 93/b7283 Bezug genommen.

### Figurenübersicht

- Fig. 1:: Transfer von DNA in K562-Zellen mittels Transfektionskomplexen, enthaltend endosomolytische Ty-Partikel, die über Biotin-Streptavidin an Polylysin gekoppelt sind
- Fig. 2:: Transfer von DNA in K562-Zellen mittels Transfektionskomplexen, enthaltend endosomolytische Ty-Partikel, die direkt an Polylysin gekoppelt sind
- Fig. 3 und 4:: Transfer von DNA in K562-Zellen mittels Transfektionskomplexen, enthaltend endosomolytische MS2-Partikel, die über Biotin-Streptavidin an Polylysin gekoppelt sind

Die Erfindung wird anhand der folgenden Beispiele illustriert:

### Beispiel 1

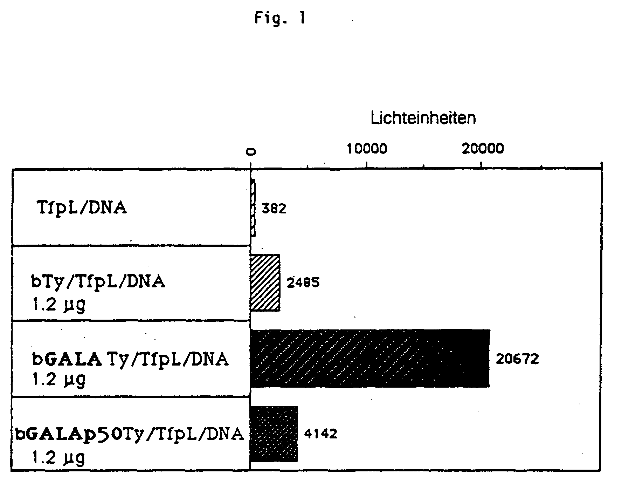

a) Konstruktion des Ty-Expressionsplasmids
   Als Ausgangsplasmid wurde das Plasmid pJef1668 verwendet. Dieses Plasmid ist abgeleitet von dem von Boeke et al., 1988, beschriebenen Plasmid pGTyH3, aus dem die zwei internen Bgl II-Fragmente entfernt worden waren. Zunächst wurde die BamHI-Stelle bei Position 2695 entfernt, indem mit BamHI geschnitten, mit Klenow aufgefüllt und religiert wurde; der erhaltene Klon wurde pJefnoBam genannt. Die PCR-Primer der Bezeichnung TyBstX.1 (SEQ ID NO:1) und Tya.2 (SEQ ID NO:2) wurden mit pJef1668 als Vorlage benutzt, um ein 870 bp Fragment (Fragment 1) zu bilden, das bei Position 1952 eine neue BamHI-Stelle enthält. Dann wurde pJefnoBam als Vorlage benutzt, um mit den Primern der Bezeichnung TyB.1 (SEQ ID NO:3) und TyB.2 (SEQ ID NO:4) das Fragment 2 erhalten. Das SalI/BstXI-Fragment aus pJef1668 wurde entfernt und durch die PCR-Fragmente 1 und 2 ersetzt. Abschließend wurde mit den komplementären Oligonukleotiden Tystop.1 (SEQ ID NO:5) und Tystop.2 (SEQ ID NO:6) eine synthetische Translationsterminationssequenz in die BamHI-Stelle eingeführt, um das Plasmid der Bezeichnung pJefTerm4 zu erhalten.
   Dadurch wurde das Einführen von DNA-Sequenzen,
   kodierend für membranaktive Peptide, die auf der Oberfläche des resultierenden TyA-Fusionsproteins exprimiert werden sollten, in die nun nur einmal vorkommende BamHI-Stelle ermöglicht. In diese Stelle wurden zwei verschiedene, für membranaktive Peptide kodierende Sequenzen eingeführt: die komplementären Oligonukleotide der Bezeichnung GALA.1 (SEQ ID NO:7) und GALA.2 (SEQ ID NO:9), kodierend für die Original-GALA-Sequenz minus Trp am N-Terminus (Subbarao et al., 1987; Parente et al., 1990) (SEQ ID NO:8); die komplementären Oligonukleotide GALAP50.1 (SEQ ID NO:10) und GALAP50.2 (SEQ ID NO:12), die für das chimäre Peptid der Bezeichnung GALAP50 (SEQ ID NO.11) kodieren. Die erhaltenen Plasmide wurden pJefGALA und pJefGALAP50 bezeichnet; die Plasmide wurden über die eingefügte Region sequenziert, um die Korrektheit der Modifikation zu bestätigen.
b) Expression von modifizierten Ty-Partikeln in Hefe
   Die Plasmide wurden unter Verwendung der Lithiumacetatmethode (Schiestl und Gietz, 1989) in den pep--Saccharomyces cerevisiae-Stamm 1268 eingeführt, womit dieser zu einer Uracil-Auxotrophie transformiert wurde (das Plasmid hat einen ura-Marker). Einzelne Klone wurden auf Uracil-Minus-Platten (pro Liter: 8 g Hefe-Stickstoffbasis, ohne Aminosäuren, 22 g Agar, 55 mg Tyrosin, 55 mg Adenin, 11 g CAA-Vitaminassay, autoklaviert, gekühlt auf 50°C, Zugabe von 100 ml 10 % Raffinose, 10 ml 0.5 % Tryptophan, 10 ml 0.5 % Leucin) selektiert und in Uracil-Minus-Medium (identisch mit der Zusammensetzung auf den Platten, ohne Agar) expandiert. Nach 24 stündigem Wachstum bei 30°C (Zelldichte ca. 108 Zellen/ml) wurde Galaktose (auf 2 %) beigegeben, um den gal4-Promotor zu induzieren, und die Zellen wurden weitere 24 h wachsen gelassen. Danach wurden die induzierten Zellen mittels Zentrifugation geerntet, in Wasser gewaschen und abschließend in 4 ml kaltem Puffer B/Mg (10 mM HEPES-KOH pH 7.8, 15 mM KCl, 5 mM MgCl₂, 3 mM DTT, 10 µg/ml Aprotinin) in 50 ml Falconröhrchen aufgenommen. Alle weiteren Schritte wurden auf Eis durchgeführt: Die Zellen wurden durch Zugabe von 5 g kalten, säuregewaschenen Glaskugeln und 5 min Vortexen, unterbrochen von 30 bis 60 sek Kühlen auf Eis, lysiert. Die Suspension wurde 5 min bei 3.000 rpm (4°C) zentrifugiert und der Überstand auf Eis gehalten (15 ml Corex-Röhrchen). Dieser Schritt wurde noch zweimal wiederholt, mit 4 ml bzw. 3 ml desselben Puffers. Das Lysat wurde abschließend 10 min bei 10.000 rpm (4°C) in einem Sorvall SS34-Rotor zentrifugiert. Portionen (2.75 ml) des Homogenats wurden dann in 3 ml Zentrifugenröhrchen überführt, dann wurden vorsichtig 250 µl 60 % Saccharose in Puffer B/EDTA (entspricht Puffer B/Mg ohne Aprotinin und MgCl₂, dafür enthaltend 10 mM EDTA) unterschichtet und bei 100.000 rpm (TLA-100.3-Rotor) 20 min lang bei 4°C zentrifugiert. Der Überstand bis zu der unscharfen ("fuzzy") Zwischenphase oberhalb des Saccharose-Polsters wurde verworfen und das Pellet nochmals in dem verbleibenden Rest plus 1 ml zusätzlichem B/EDTA-Puffer aufgenommen, dieses Material in ein frisches Zentrifugenröhrchen gegeben und auf 1.5 ml 35 % Saccharose / B/EDTA-Puffer geschichtet. Zur Unterschichtung wurden 250 µl desselben Materials wie beim ersten Durchgang verwendet. Die Zentrifugation wurde genauso durchgeführt wie beim ersten Mal; das auf diese Weise aus mehreren Doppelzentrifugationen gewonnene Material wurde zusammengeführt, mit 400 µl 60 % Saccharaose / B/EDTA-Puffer unterschichtet und 1 h in einem SW41-Rotor bei 4°C und 39 rpm zentrifugiert. Die erhaltenen Pellets wurden in ca. 1.5 ml B/EDTA-Puffer aufgenommen, womit die Saccharose auf weniger als 12.5 % verdünnt wurde, und anschließend wurden die 800 µl Proben des Materials auf einem linearen 15 bis 50 % Saccharosegradienten (13 ml) in Puffer B/EDTA (25.000 rpm, 3 h, 4°C, SW41-Rotor) fraktioniert. Die Fraktionen wurden mittels SDS/PAGE auf ihren Proteingehalt untersucht und die das TyA-Protein enthaltenden Fraktionen vereinigt.
c) Modifikation der Ty-Partikel
   i) Biotinylierung
      Die Biotinylierung der Ty-Partikel zwecks Bindung an Streptavidin-Polylysin wurde im wesentlichen durchgeführt, wie in der WO 93/07283 u.a. für Adenovirus beschrieben, wobei NHS-LC-Biotin (Pierce Kat.Nr. 21335) in 10 mM HEPES pH 7.9 zu 1 mM aufgelöst und die Biotinlösung der Ty-Partikellösung (10 µl pro ml) beigegeben wurde. Nach 3 stündiger Reaktion bei Raumtemperatur wurde die Probe ausgiebig gegen HBS/40 % Glycerin bei 4°C dialysiert, um das nicht-reagierte Biotin zu entfernen.
   ii) Kopplung von Polylysin mittels Transglutaminase
      Aufgrund der verwendeten Klonierungsmethode zur Herstellung des Plasmids pJefGALA enthält die GALA-Sequenz einen Lysinrest; die Anfügung der Biotingruppe und die anschließende Bindung von Streptavidin könnte die von dieser Sequenz erwartete Membranwechselwirkung beeinträchtigen. Es wurde daher alternativ die direkte Kopplung von Polylysin an das Ty-Partikel mittels Transglutaminase durchgeführt.
      Die Reaktion wurde im wesentlichen durchgeführt, wie in der WO 93/07283 beschrieben: Proben der gereinigten TyGALA-Partikel (500 µl, 0.2 mg/ml) in 100 mM HEPES pH 7.9, 2 mM DTT, 10 mM CaCl₂, wurden mit 1 nmol Meerschweinchen-Lebertransglutaminase (Sigma) und 50 µl Polylysin (Kettenlänge 200; 1 mg/ml) 2 h lang bei 37°C inkubiert. Die Polylysin-modifizierten Ty-Partikel wurden von freiem Polylysin gereinigt, indem die Probe in HBS verdünnt, mit einem 60 % Saccharose-Polster unterschichtet und 40 min lang in einem TLA-100.3-Rotor zentrifugiert wurde. Das zentrifugierte Material wurde in HBS/40 % Glycerin über Nacht bei 4°C aufgenommen und direkt für die DNA-Tränsferversuche verwendet.
d) Herstellung von Humantransferrin-Polylysin
   Es wurde die von Wagner et al., 1991b, beschriebene Methode verwendet, bei der die Kopplung von Polylysin an die Kohlenhydratseitenketten des Transferrins erfolgt.
   Eine Lösung von 91 mg (1.14 µmol) humanem Transferrin (eisenfrei, Biotest Pharma) in 1.4 ml 30 mM Natriumacetatpuffer, pH 5, wurde auf 0°C gekühlt und 34 µl 30 mM Natriumacetatpuffer pH 5, enthaltend 0.73 mg (3.4 µmol) Natriumperiodat hinzugefügt. Die Mischung wurde im Eisbad 90 min lang im Dunklen stehen gelassen. Zur Entfernung der niedermolekularen Produkte wurde eine Gelfiltration durchgeführt (Sephadex G-25, Pharmacia), die eine Lösung mit einem Gehalt von ca. 82 mg (2 ml) oxidiertem Transferrin (Messung durch Ninhydrinassay) ergab. (Um die oxidierte Form nachzuweisen, die Aldehyde enthält und bei Färbung mit Anisaldehyd eine Farbreaktion gibt, wurden die Proben auf eine Silikagel-Dünnschichtplatte getropft, getrocknet und die Platten in p-Anisaldehyd/Schwefelsäure/Ethanol (1/1/18) getaucht, getrocknet und erhitzt.) Die modifizierte Transferrin-Lösung wurde rasch (innerhalb 10 bis 15 min) einer Lösung, enthaltend 1.0 µmol Poly(L)Lysin mit einer durchschnittlichen Kettenlänge von 250 Lysinmonomeren in 0.9 ml Wasser hinzugefügt. Der pH-Wert der Lösung wurde durch Zusatz von 0.3 ml 2 M HEPES pH 7.9 auf pH 7.7 eingestellt. Zu der Mischung wurden in Abständen von je 1 h 4 Portionen von je 8 mg (126.3 µmol) Natriumcyanoborhydrid hinzugefügt. Nach 17 h wurden 1 ml 5 M Natriumchlorid und 5.8 ml Wasser zugefügt, um die Lösung auf eine Gesamtkonzentration von ca. 0.5 M zu bringen. Die Reaktionsmischung wurde auf eine Kationenaustauschsäule (Bio-Rad MacroprephigS in Säule HR 10/10) aufgebracht und mit einem Salzgradienten von 0.5 M bis 3.0 M Natriumchlorid mit einem konstanten Gehalt von 20 mM HEPES pH 7.3 fraktioniert. Die hohe Salzkonzentration beim Laden der Säule und ab Beginn des Gradienten war wesentlich für die Gewinnung der Polykation-Konjugate. Die Hauptmenge von Konjugat eluierte bei einer Salzkonzentration zwischen 2.1 M und 2.6 M und wurde gepoolt. Diese Fraktionen ergaben (in der Reihenfolge ihrer Elution) nach einmaliger Dialyse gegen 2 1 HBS (20 mM HEPES pH 7.3 150 mM NaCl) eine Hauptfraktion (TfpL250) mit einem Gehalt an 54 mg (0.67 µmol) Transferrin, modifiziert mit 39.4 mg = 0.76 µmol Polylysin. Die Transferrin-Konjugate wurden, sofern sie nicht sofort verwendet wurden, nach Schockgefrieren in flüssigem Stickstoff bei -20°C in eisenfreier Form gelagert. Der Eiseneinbau wurde durch Hinzufügen von 1.25 µl 10 mM Eisen(III)citratpuffer (enthaltend 200 mM Citrat, durch Zugabe von Natriumbicarbonat auf einen pH-Wert von 7.8 eingestellt) pro mg Transferrin-Gehalt vorgenommen. Die eisenhaltigen Konjugate wurden vor ihrer Verwendung für die DNA-Komplexbildung in kleine Aliquots aufgeteilt, schockgefroren in flüssigem Stickstoff oder Trockeneis/Ethanol und bei -20°C aufbewahrt. (Diese Maßnahme erwies sich als zweckmäßig, nachdem sich gezeigt hatte, daß mehrmaliges Auftauen und Einfrieren den Verderb der Konjugate zur Folge hat.)
e) Gentransfer in K562-Zellen mittels Transfektionskomplexen, die endosomolytische Ty-Partikel enthalten
   i) Verwendung von Ty-Partikeln, die über Biotin-Streptavidin mit Polylysin konjugiert sind Transfektionskomplexe, enthaltend biotinyliertes Wildtyp Ty-, TyGALA- oder TyP50-Partikel wurden wie folgt hergestellt: Die in Fig. 1 angegebenen Mengen an biotinylierten Ty-Partikeln wurden in 150 µl HBS verdünnt und mit 150 µl HBS, enthaltend 1 µg Streptavidin-Polylysin, 30 min lang bei Raumtemperatur gemischt. Dann wurde ein 100 µl Aliquot HBS, enthaltend 6 µg pCMVL-DNA beigegeben und die Mischung 30 min bei Raumtemperatur stehengelassen. Abschließend wurde ein 100 µl Aliquot HBS, enthaltend 5.6 µg Transferrin-Polylysin hinzugefügt. Zur Kontrolle wurden Komplexe aus DNA, Streptavidin-Polylysin und Transferrin-Polylysin eingesetzt. Diese Komplexe wurden auf 500.000 Deferrioxamin-behandelte K562-Zellen aufgebracht, wie in der WO 93/07283 beschrieben. 24 h später wurden die Zellen geerntet, Zellextrakte hergestellt und auf Luciferaseaktivität untersucht. Es zeigte sich, daß die als Kontrolle eingesetzten Komplexe nicht funktionieren, daß das Wildtyp Ty-Partikel eine geringfügige Zunahme der Luciferaseaktivität hervorruft und daß der Gehalt an biotinyliertem Ty, das mit dem membranaktiven Peptid GALA modifiziert ist, in den Komplexen eine ca. 10fache Zunahme des DNA-Transfers gegenüber den Wildtyp-Ty-Partikeln bewirkt. Die mit GALAP50 modifizierten Ty-Partikel induzierten eine geringe Zunahme des DNA-Transports. (In allen Figuren sind die Mittelwerte aus zwei Transfektionen angegeben.)
   ii) Verwendung von Ty-Partikeln, die direkt an Polylysin gekoppelt sind

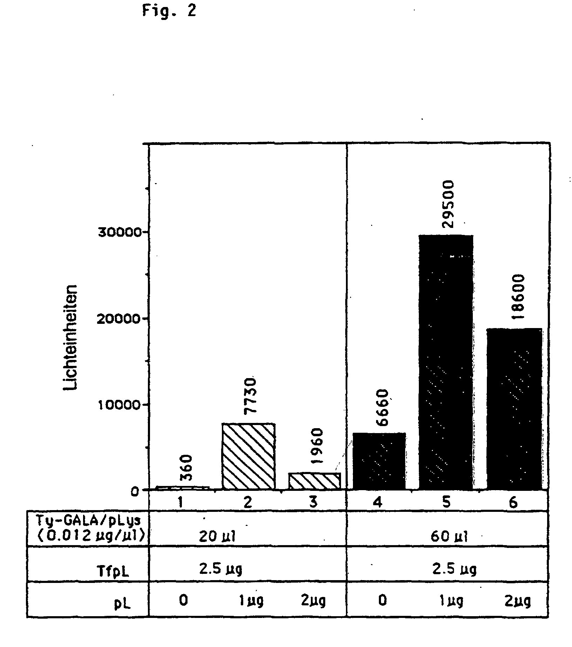

   Die Polylysin-modifizierten TyGALA-Partikel wurden in DNA-Komplexe inkorporiert, wie unter i) angegeben, mit dem Unterschied, daß die Inkubation mit Streptavidin-Polylysin unterblieb und die Transferrin-Polylysin/HBS-Lösung zusätzlich die in Fig. 2 angegebenen Mengen an freiem Polylysin enthielten, um die vollständige Kondensation der DNA zu gewährleisten. Die Komplexe wurden auf Deferrioxamin-stimulierte Zellen aufgebracht, 24 h später wurden Extrakte hergestellt und auf Luciferaseaktivität untersucht. Es zeigte sich, daß die mit den Transglutaminase-gekoppelten Ty-Polylysin-Konjugaten erzielten absoluten Expressionswerte diejenigen nicht überschritten, die mit den Biotin-Streptavidin-gekoppelten Konjugaten erzielt wurden.

### Beispiel 2

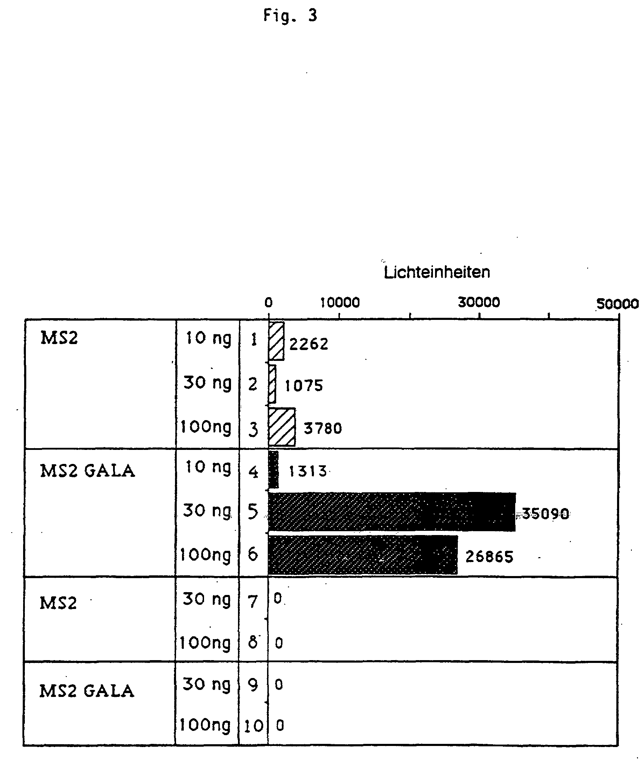

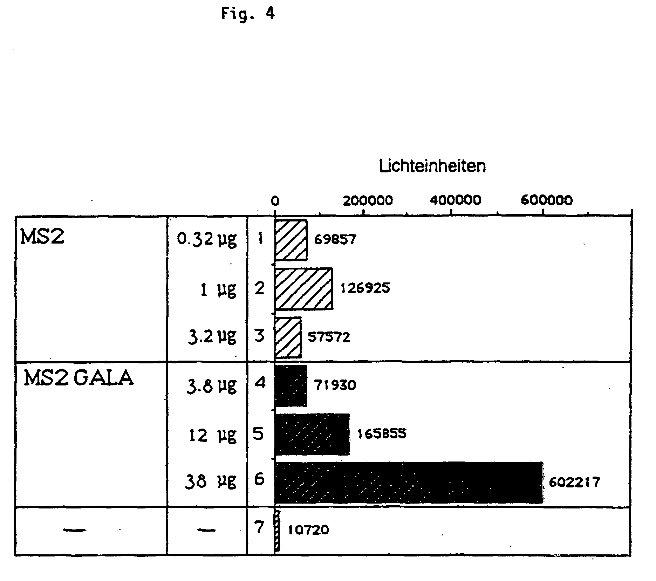

a) Konstruktion von MS2-Kapsid-Expressionsplasmiden
   Das Plasmid der Bezeichnung pPLaACR26 (Remaut et al., 1981), das die für MS2 kodierende Sequenz enthält, wurde von der LMBP Culture Collection Laboratory of Molecular Biology, Universität Gent, Belgien, bezogen. Um die MS2-Kapsid-Sequenz als BglII-Fragment zu isolieren und die Sequenz zwecks Bildung einer nur einmal vorkommenden BamHI-Stelle bei den Nukleotiden, die für Aminosäure 15 kodieren, zu mutieren, wurde die PCR-Methode eingesetzt. Das PCR-Fragment wurde mittels Gelelektrophorese gereinigt und in das BamHI geschnittene Plasmid pETH2a hineinligiert (pETH2a ist der T7-Expressionsvektor pET2a (Studier et al., 1990), in dem die kleine NdeI/BamHI-Stelle durch die für Polyhistidin (SEQ ID NO:14) kodierenden komplementären Oligonukleotide A (SEQ ID NO:13) und B (SEQ ID NO:15) ersetzt wurde). Ein Klon der Bezeichnung pMS2WT9, der das Insert in der richtigen Orientierung enthielt, wurde isoliert; das Vorhandensein der richtigen Sequenz wurde durch Sequenzieren bestätigt.
   Das Plasmid pMS2GALA4 wurde hergestellt, indem die für GALA kodierenden komplementären Oligonukleotide GALAMS1 und GALAMS2 in die einzige BamHI-Stelle von pMS2WT9 eingesetzt wurden. GALAMS1 ist identisch mit GALA.1, mit dem Unterschied, daß T bei Position 5 entfernt wurde. GALAMS2 ist identisch mit GALA.2, mit dem Unterschied, daß das endständige A entfernt wurde. Dadurch wird die GALA-Sequenz in den richtigen Leserahmen für die Expression des modifizierten MS2-Kapsids gesetzt. Das Vorhandensein des richtigen DNA-Inserts in der richtigen Orientierung wurde wieder mittels DNA-Sequenzierung bestätigt.
b) Expression von MS2-Kapsiden
   Die Plasmide pMS2WT9 und pMS2GALA4 wurden in den T7-Expressionsbakterienstamm BL21 (DE3)(Studier et al., 1990; bezogen von Novagen) transformiert, Einzelkolonien selektioniert und in 1 Liter Kulturen gezüchtet (OD₆₀₀ = 0.7), daraufhin wurde IPTG (auf 1 mM) hinzugefügt, um die T7-Polymeraseexpression und anschließend die Expression der MS2- und MS2GALA-Proteine zu induzieren. Nach 4 h bei 37°C wurden die Zellen mittels Zentrifugation geerntet und die Bakterienzellpellets in 6 M Guanidinhydrochlorid, 0.1 M Natriumphosphat, 10 mM β-Mercaptoethanol und 10 mM Tris, pH 8.0 (Puffer A) unter einstündigem Rühren bei Zimmertemperatur lysiert. Das Lysat wurde mittels Zentrifugation bei 17.000 rpm (Sorval SS34-Rotor) geklärt und der Überstand zwecks Ernte der Polyhistidin-markierten Proteine über eine 3 ml Nickel-Chelat NTA-Sepharosesäule, equilibriert mit Puffer A, geschickt. Die Säulen wurden eluiert mit 10 Säulenvolumina Puffer A; 5 Säulenvolumina 6 M Harnstoff, 100 mM Natriumphosphat, 10 mM Tris, pH 6.5; 5 Säulenvolumina 6 M Harnstoff, 100 mM Natriumphosphat, 10 mM Tris, pH 5.7. Abschließend wurden 5 Säulenvolumina 0.2 N Essigsäure/6 M Guanidinhydrochlorid eingesetzt, um die MS2- und MS2GALA-Proteine zu eluieren. Um das Denaturierungsmittel zu entfernen, wurden die Eluate über kleine Gelfiltrationssäulen (Pharmacia "Nick Columns" oder Pharmacia PD-10 Säulen), equilibriert mit 100 mM DTT, 40 mM HEPES, pH 7.4, geschickt.
c) Biotinylierung von MS2-Kapsiden
   Die unter b) erhaltenen Kapsid-Proteine wurden biotinyliert wie die Ty-Partikel im vorangegangenen Beispiel, ausgiebig gegen 40 mM HEPES pH 7.4 dialysiert und bei 4°C gelagert.
d) Gentransfer in K562-Zellen mittels Transfektionskomplexen, die endosomolytische MS2-Partikel enthalten
   i) Transfektionskomplexe, enthaltend biotinyliertes MS2-Partikel wurden wie folgt hergestellt: Die in Fig. 3 angegebenen Mengen an biotinylierten MS2-Partikeln (Wildtyp-MS2 und MS2-GALA in biotinylierter (Proben 1-6) und in nicht-modifizierter (Proben 7-9) Form) wurden in 150 µl HBS verdünnt und mit 150 µl HBS, enthaltend 1 µg Streptavidin-Polylysin, 30 min lang bei Raumtemperatur gemischt. Dann wurde ein 100 µl Aliquot HBS, enthaltend 6 µg pCMVL-DNA beigegeben und die Mischung 30 min bei Raumtemperatur stehengelassen. Abschließend wurde ein 100 µl Aliquot HBS, enthaltend 5.6 µg Transferrin-Polylysin (s. Beispiel 1) hinzugefügt. Diese Komplexe wurden auf 500.000 Deferrioxamin-behandelte K562-Zellen aufgebracht, wie in der WO 93/07283 beschrieben. 24 h später wurden die Zellen geerntet, Zellextrakte hergestellt und auf Luciferaseaktivität untersucht.
   ii) Es wurde eine zweite Versuchsreihe mit biotinyliertem Wildtyp-MS2 und MS2-GALA, wie unter i) durchgeführt, mit dem Unterschied, daß größere Mengen (wie in Fig. 4 angegeben) an biotinylierten MS2-Partikeln verwendet wurden. Es zeigte sich, daß die biotinylierten Wildtyp-Partikel (Proben 1-3) nur eine geringfügige Verstärkung des DNA-Transports gegenüber dem Background bewirkten, während die Gegenwart von biotinylierten MS2-GALA-Partikeln (Proben 4-6) den DNA-Transport entsprechend 600.000 Lichteinheiten stimulierte. (Probe 7 enthielt keine MS2-Partikel.)

### Literatur

Ackerman, H.W. und DuBow, M.S., 1987, Viruses of Prokaryotes, Vol. II, pp. 171-218, CRC Press, Boca Raton, Florida.
Boeke, J., Eichinger, D., Castrillon, D. und Fink, G., 1988, Mol. Cell. Biol. 8, 1432-1442.
Brown, C.S., van Lent, W.M., Vlak, J.M. und Spaan, W.J.M., 1991, J. Virol. 65, 2702-2706.
Burns, N., Saibil, H., White, N., Pardon, J., Timmons, R., Richardson, S., Richards, B., Adams, S., Kingsman, S. und Kingsman, A., 1992, EMBO J. 11, 1155-1164.
Christensen, J., Storgaard, T., Bloch, B., Alexandersen, S. und Aasted, B., 1993, J. Virol. 67, 229-238.
Cotten, M., Wagner, E., Zatloukal, K., Phillips, S., Curiel, D. und Birnstiel, M.L., 1992, Proc.Natl.Acad.Sci. USA 89, 6094-6098.
Cotten, M., Wagner, E. und Birnstiel, M.L., 1993a, Methods Enzymol. 217, 618-644.
Cotten, M., Wagner, E., Zatloukal, K. und Birnstiel, M.L., 1993b, J. Virol. 67, 3777-3785.
Curiel, D.T., Agarwal, S., Wagner, E. und Cotten, M., 1991, Proc.Natl.Acad.Sci. USA 88, 8850-8854.
Daniell, E., 1976, J. Virol. 19, 685-708.
Defer, C., Belin, M., Caillet-Boudin, M. und Boulanger, P., 1990, J. Virol. 64, 3661-3673.
Emr, S.D., 1990, Methods Enzymology 185, 231-233.
Fiers, W., Contreras, R., Duerinck, F., Haegeman, G., Iserentant, D., Merregaert, J., Minjou, W., Molemans, F., Raeymaekers, A., Van den Berghe, A., Volckaert, G. und Ysebaert, M., 1976, Nature 260, 500-507.
Icho, T. und Wickner, R.B., 1989, J. Biol. Chem. 264, 6716-6723.
Jiang, X., Wang, M., Graham, D.Y. und Estes, M.K., 1992, J. Virol. 66, 6527-6532.
Jung, et al., 1981, Biochem. Res. Commun. 101, 599.
Kay, R., Takei, F. und Humphries, R.K., 1990, J. Immunology 145, 1952-1959.
Kingsman, A.J., Adams, S.E., Burns, N.R. und Kingsman, S.M., 1991, Trends in Biotechnology 9, 303-309.
Mastico, R.A., Talbot, S.J. und Stockley, P.G., 1993, J. Gen. Virology 74, 541-548.
O'Neil, K.T., Hoess, R.H., Jackson, S.A., Ramachandran, N.S., Mousa, S.A. und DeGrado, W.F., 1992, Proteins 14, 509-515.
O'Reilly, D.R., Miller, L.K. und Luckow, V.A., 1992, Baculovirus expression vectors. W.H. Freeman & Co. New York.
Parente, R.A., Nir, S. und Szoka, F.C., 1990, Biochemistry 29, 8720-8728.
Pierschbacher, M.D. und Ruoslahti, E., 1984, Nature 309, 30-33.
Pierschbacher, M.D. und Ruoslahti, E., 1987, J. Biol. Chem. 262, 17294-17298.
Remaut, E., Stanssens, P. und Fiers W., 1981, Gene 15, 81-93.
Ruffing, M., Zentgraf, H. und Kleinschmidt, J.A., 1992, J. Virol. 66, 6922-6930.
Schiestl, R.H. und Gietz, R.D., 1989, Current Genetics 16, 339-346.
Schneeman, A., Dasgupta, R., Johnson, J.E. und Rueckert, R.R., 1993, J. Virol. 67, 2756-2763.
Seth, P., FitzGerald, D., Ginsberg, H., Willingham, M. und Pastan, I., 1984, Mol. Cell. Biol. 4, 1528-1533.
Stewart, P.L., Fuller, S.D. und Burnett, R.M., 1993, EMBO J. 12, 2589-2599.
Studier, W., Rosenberg, A.H., Dunn, J.J. und Dubendorff, J.W.. 1990, Methods Enzymol. 185, 60-89.
Subbarao, N.K., Parente, R.A., Szoka, F.C., Nadasdi, L. und Pongracz, K., 1987, J. Biol. Chem. 26, 2964-2972.
Torres, B.A., Griggs, N.D. und Johnson, H.M., 1993, Nature 364, 152-154.
Urakawa, T., Ferguson, M., Minor, P.D., Cooper, J., Sullivan, M., Almond, J.W. und Bishop, D.H.L., 1989, J. Gen. Virol. 70, 1453-1463.
Valegard, K., Lijas, L., Fridborg, K. und Unge, T., 1990, Nature 345, 36-41.
Wagner, E., Cotten, M., Foisner, R. und Birnstiel, M.L., 1991a, Proc.Natl.Acad.Sci. USA 88, 4255-4259.
Wagner, E., Cotten, M., Mechtler, K., Kirlappos, H. und Birnstiel, M.L., 1991b, Bioconjugate Chemistry 2, 226-231.
Wagner, E., Zatloukal, K., Cotten, M., Kirlappos, H., Mechtler, K., Curiel, D. und Birnstiel, M.L., 1992a, Proc.Natl.Acad.Sci. USA 89, 6099-6103.
Wagner, E., Plank, C., Zatloukal, K., Cotten, M. und Birnstiel, M.L., 1992b, Proc.Natl.Acad.Sci. USA 89, 7934-7938.
Weber, J., 1976, J. Virol. 17, 462-471.
Wickner, R.B., 1993, J. Biol. Chem. 268, 3797-3800.
Wickham, T.J., Mathias, P., Cheresh, D.A. und Nemerow, G.R., 1993, Cell 73, 309-319.
Wu, G.Y, und Wu, C.H., 1987, J. Biol. Chem. 262, 4429-4432.
Zatloukal, K., Wagner, E., Cotten, M., Phillips, St., Plank, C., Steinlein, P., Curiel, D. und Birnstiel, M.L., 1992, Annals New York Academy of Sciences 660, 136-153.

## Patentansprüche

1. Endosomolytisch wirksames virusähnliches Partikel, **dadurch gekennzeichnet, daß** es aus Einheiten von Kapsidproteinen, abgeleitet von Viren oder virusähnlichen Teilchen, zusammengesetzt ist, die mit einer membranaktiven peptidischen Sequenz modifiziert sind.

2. Partikel nach Anspruch 1, **dadurch gekennzeichnet, daß** es ein modifiziertes Hefe-Ty-Partikel ist, das aus Einheiten von TyA-Protein zusammengesetzt ist, die mit einer membranaktiven Peptidsequenz modifiziert sind.

3. Partikel nach Anspruch 2, **dadurch gekennzeichnet, daß** das Ty-Partikel mit der Peptidsequenz Glu Ala Ala Leu Ala Glu Ala Leu Ala Glu Ala Leu Ala Glu His Leu Ala Glu Ala Leu Ala Glu Ala Leu Glu Ala Leu Ala Ala modifiziert ist, die sich am Carboxy-Terminus des TyA-Proteins befindet.

4. Partikel nach Anspruch 2, **dadurch gekennzeichnet, daß** das Ty-Partikel mit der Peptidsequenz Gly Leu Phe Glu Ala Ile Glu Gly Phe Ile Glu Asn Gly Trp Glu Gly Leu Ala Glu Ala Leu Ala Glu Ala Leu Glu Ala Leu Ala Ala Gly Gly Ser modifiziert ist, die sich am Carboxy-Terminus des TyA-Proteins befindet.

5. Partikel nach Anspruch 1, **dadurch gekennzeichnet, daß** es ein modifiziertes MS2-Partikel ist, das aus Einheiten des MS2-Kapsidproteins zusammengesetzt ist, die mit einer membranaktiven Peptidsequenz modifiziert sind.

6. Partikel nach Anspruch 5, **dadurch gekennzeichnet, daß** das membranaktive Peptid in der β-Haarnadelschleifenregion zwischen Aminosäure 11 (Asp) und Aminosäure 17 (Asp), insbesondere zwischen Aminosäure 14 (Gly) und 15 (Thr), des MS2-Kapsidproteins eingefügt ist.

7. Partikel nach Anspruch 5 oder 6, **dadurch gekennzeichnet, daß** es mit der Peptidsequenz Glu Ala Ala Leu Ala Glu Ala Leu Ala Glu Ala Leu Ala Glu His Leu Ala Glu Ala Leu Ala Glu Ala Leu Glu Ala Leu Ala Ala modifiziert ist.

8. Partikel nach einem der Ansprüche 1 bis 7, **dadurch gekennzeichnet, daß** es außerdem eine Nukleinsäure-bindende Domäne aufweist.

9. Partikel nach Anspruch 8, **dadurch gekennzeichnet, daß** die Nukleinsäure-bindende Domäne Polylysin ist.

10. Partikel nach Anspruch 9, **dadurch gekennzeichnet, daß** es direkt an Polylysin gebunden ist.

11. Partikel nach einem der Ansprüche 1 bis 8, **dadurch gekennzeichnet, daß** es außerdem eine Peptidsequenz aufweist, die die Funktion eines Liganden für eine höhere eukaryotische Zelle hat.

12. Verfahren zur Herstellung von Partikeln nach Anspruch 1, **dadurch gekennzeichnet, daß** man eine für ein Kapsidprotein von Viren oder virusähnlichen Teilchen kodierende DNA, die mit einer für ein membranaktives Peptid kodierenden Sequenz modifiziert ist, exprimiert und das erhaltene Kapsid erntet oder erforderlichenfalls die Kapsidproteinmonomeren zu Kapsidstrukturen assoziieren läßt.

13. Verfahren zur Herstellung von Hefe-Ty-Partikeln nach Anspruch 2, **dadurch gekennzeichnet, daß** man eine DNA, kodierend für das TyA-Protein, das am Carboxy-Terminus ein membranaktives Peptid enthält, in Hefezellen exprimiert, die Zellen aufschließt und die Partikel erntet.

14. Verfahren zur Herstellung von MS2-Partikeln nach Anspruch 5, **dadurch gekennzeichnet, daß** man eine DNA, kodierend für das MS2-Kapsidprotein, die eine für ein membranaktives Peptid kodierene Sequenz eingefügt enthält, exprimiert, das erhaltene modifizierte Kapsidprotein denaturiert und nach Entfernung des Denaturierungsmittels assoziieren läßt.

15. Zusammensetzung für den Transport von Nukleinsäure in die höhere eukaryotische Zelle, enthaltend ein endosomolytisches Mittel, **dadurch gekennzeichnet, daß** das endosomolytische Mittel ein endosomolytisch wirksames virusähnliches Partikel nach einem der Ansprüche 7 bis 11 ist.

16. Zusammensetzung nach Anspruch 15, daß sie außerdem ein Konjugat aus einer Nukleinsäure-bindenden Substanz und einem Internalisierungsfaktor für die Zelle enthält.

17. Zusammensetzung nach Anspruch 16, **dadurch gekennzeichnet**, sie ein Transferrin-Polylysin-Konjugat enthält.

## Claims

1. Endosomolytically active virus-like particle, **characterised in that** it is made up of units of capsid proteins derived from viruses or virus-like particles which are modified with a membrane-active peptide sequence.

2. Particle according to claim 1, **characterised in that** it is a modified yeast-Ty particle which is made up of units of TyA protein modified with a membrane-active peptide sequence.

3. Particle according to claim 2, **characterised in that** the Ty particle is modified with the peptide sequence Glu Ala Ala Leu Ala Glu Ala Leu Ala Glu Ala Leu Ala Glu His Leu Ala Glu Ala Leu Ala Glu Ala Leu Glu Ala Leu Ala Ala which is located at the carboxy terminus of the TyA protein.

4. Particle according to claim 2, **characterised in that** the Ty particle is modified with the peptide sequence Gly Leu Phe Glu Ala Ile Glu Gly Phe Ile Glu Asn Gly Trp Glu Gly Leu Ala Glu Ala Leu Ala Glu Ala Leu Glu Ala Leu Ala Ala Gly Gly Ser, which is located at the carboxy terminus of the TyA protein.

5. Particle according to claim 1, **characterised in that** it is a modified MS2 particle which made up of units of the MS2 capsid protein modified with a membrane-active peptide sequence.

6. Particle according to claim 5, **characterised in that** the membrane-active peptide is inserted in the β-hairpin loop region between amino acid 11 (Asp) and amino acid 17 (Asp), particularly between amino acid 14 (Gly) and 15 (Thr) of the MS2 capsid protein.

7. Particle according to claim 5 or 6, **characterised in that** it is modified with the peptide sequence Glu Ala Ala Leu Ala Glu Ala Leu Ala Glu Ala Leu Ala Glu His Leu Ala Glu Ala Leu Ala Glu Ala Leu Glu Ala Leu Ala Ala.

8. Particle according to one of claims 1 to 7, **characterised in that** it also has a nucleic acid-binding domain.

9. Particle according to claim 8, **characterised in that** the nucleic acid-binding domain is polylysine.

10. Particle according to claim 9, **characterised in that** it is bound directly to polylysine.

11. Particle according to one of claims 1 to 8, **characterised in that** it also has a peptide sequence which has the function of a ligand for a higher eukaryotic cell.

12. Process for preparing particles according to claim 1, **characterised in that** a DNA coding for a capsid protein of viruses or virus-like particles, which is modified with a sequence coding for a membrane-active peptide, is expressed and the resulting capsid is harvested or, if necessary, the capsid protein monomers are allowed to associate into capsid structures.

13. Process for preparing yeast-Ty particles according to claim 2, **characterised in that** a DNA coding for the TyA protein which contains a membrane-active peptide at the carboxy terminus is expressed in yeast cells, the cells are opened up and the particles are harvested.

14. Process for preparing MS2 particles according to claim 5, **characterised in that** a DNA coding for the MS2 capsid protein which contains a sequence inserted therein coding for a membrane-active peptide is expressed, the resulting modified capsid protein is denatured and after removal of the denaturing agent it is allowed to associate.

15. Composition for transporting nucleic acid into the higher eukaryotic cell, containing an endosomolytic agent, **characterised in that** the endosomolytic agent is an endosomolytically active virus-like particle according to one of claims 7 to 11.

16. Composition according to claim 15 **characterised in that** it also contains a conjugate of a nucleic acid-binding substance and an internalising factor for the cell.

17. Composition according to claim 16, **characterised in that** it contains a transferrin-polylysine conjugate.

## Revendications

1. Particule analogue à un virus endosomolytiquement efficace **caractérisée en ce qu'**elle est composée d'unités de protéines de capside issues de virus ou de particules analogues à des virus qui sont modifiées avec une séquence peptidique à activité membranaire.

2. Particule selon la revendication 1 **caractérisée en ce qu'**il s'agit d'une particule Ty de levure modifiée qui est composée d'unités de protéine TyA qui sont modifiées avec une séquence peptidique à activité membranaire.

3. Particule selon la revendication 2 **caractérisée en ce que** la particule Ty est modifiée avec la séquence peptidique qui se trouve à l'extrémité carboxy-terminale de la protéine TyA.

4. Particule selon la revendication 2 **caractérisée en ce que** la particule Ty est modifiée avec la séquence peptidique qui se trouve à l'extrémité carboxy-terminale de la protéine TyA.

5. Particule selon la revendication 1 **caractérisée en ce qu'**il s'agit d'une particule MS2 modifiée qui est composée d'unités de protéine de capside MS2 qui sont modifiées avec une séquence peptidique à activité membranaire.

6. Particule selon la revendication 5 **caractérisée en ce que** le peptide à activité membranaire est inséré dans la région de boucle en épingle à cheveux β entre l'aminoacide 11 (Asp) et l'aminoacide 17 (Asp), en particulier entre les aminoacides 14 (Gly) et 15 (Thr), de la protéine de capside MS2.

7. Particule selon la revendication 5 ou 6 **caractérisée en ce qu'**elle est modifiée avec la séquence peptidique

8. Particule selon l'une des revendications 1 à 7 **caractérisée en ce qu'**elle comprend en outre un domaine liant les acides nucléiques.

9. Particule selon la revendication 8 **caractérisée en ce que** le domaine liant les acides nucléiques est la polylysine.

10. Particule selon la revendication 9 **caractérisée en ce qu'**elle est liée directement à la polylysine.

11. Particule selon l'une des revendications 1 à 8 **caractérisée en ce qu'**elle comprend en outre une séquence peptidique qui a la fonction d'un ligand pour une cellule eucaryote supérieure.

12. Procédé de production de particules selon la revendication 1 **caractérisé en ce que** l'on exprime un ADN codant une protéine de capside de virus ou de particules analogues à des virus qui est modifié avec une séquence codant un peptide à activité membranaire et on récolte la capside obtenue ou, si nécessaire, on fait s'associer en structures de capside les monomères de protéine de capside.

13. Procédé de production de particules Ty de levure selon la revendication 2 **caractérisé en ce que** l'on exprime dans des cellules de levure un ADN codant la protéine TyA qui contient à l'extrémité carboxy-terminale un peptide à activité membranaire, on lyse les cellules et on récolte les particules.

14. Procédé de production de particules MS2 selon la revendication 5 **caractérisé en ce que** l'on exprime un ADN codant la protéine de capside MS2 qui contient insérée une séquence codant un peptide à activité membranaire, on dénature la protéine de capside modifiée obtenue et on la fait s'associer après élimination de l'agent dénaturant.

15. Composition pour le transport d'acide nucléique dans la cellule eucaryote supérieure contenant un agent endosomolytique **caractérisée en ce que** l'agent endosomolytique est une particule analogue à un virus endosomolytiquement efficace selon l'une des revendications 7 à 11.

16. Composition selon la revendication 15 **caractérisée en ce qu'**elle contient en outre un conjugué d'une substance liant les acides nucléiques et d'un facteur d'internalisation pour la cellule.

17. Composition selon la revendication 16 **caractérisée en ce qu'**elle contient un conjugué transferrine-polylysine.


### SEQUENZPROTOKOLL

(1) ALLGEMEINE INFORMATION:
   (i) ANMELDER:
      (A) NAME: Boehringer Ingelheim International GmbH
      (B) STRASSE: Binger Strasse 173
      (C) ORT: Ingelheim am Rhein
      (E) LAND: BRD
      (F) POSTLEITZAHL: 55216
      (G) TELEPHON: 06132/772282
      (H) TELEFAX: 06132/774377
      (I) TELEX: 4187910 bi d
   (ii) ANMELDETITEL: Endosomolytisch wirksame Partikel
   (iii) ANZAHL DER SEQUENZEN: 15
   (iv) COMPUTER-LESBARE FORM:
      (A) DATENTRÄGER: Floppy disk
      (B) COMPUTER: IBM PC compatible
      (C) BETRIEBSSYSTEM: PC-DOS/MS-DOS
      (D) SOFTWARE: Patent In Release #1.0, Version #1.25 (EPA)
(2) INFORMATION ZU SEQ ID NO: 1:
   (i) SEQUENZ CHARAKTERISTIKA:
      (A) LÄNGE: 34 Basenpaare
      (B) ART: Nukleinsäure
      (C) STRANGFORM: Einzel
      (D) TOPOLOGIE: linear
   (ii) ART DES MOLEKÜLS: synthetisches Oligodesoxyribonukleotid
   (ix) MERKMALE:
      (A) NAME/SCHLÜSSEL: misc_feature
      (B) LAGE: 1..34
   (xi) SEQUENZBESCHREIBUNG: SEQ ID NO: 1:
   GACCCAAAAC CAAGCCAATC CATCTGGTTG GTCA 34
(2) INFORMATION ZU SEQ ID NO: 2:
   (i) SEQUENZ CHARAKTERISTIKA:
      (A) LÄNGE: 33 Basenpaare
      (B) ART: Nukleinsäure
      (C) STRANGFORM: Einzel
      (D) TOPOLOGIE: linear
   (ii) ART DES MOLEKÜLS: synthetisches Oligodesoxyribonukleotid
   (ix) MERKMALE:
      (A) NAME/SCHLÜSSEL: misc_feature
      (B) LAGE: 1..33
   (xi) SEQUENZBESCHREIBUNG: SEQ ID NO: 2:
   TGTAACTGGA TCCCCTTTGG GTTTGGTTGT ATT 33
(2) INFORMATION ZU SEQ ID NO: 3:
   (i) SEQUENZ CHARAKTERISTIKA:
      (A) LÄNGE: 35 Basenpaare
      (B) ART: Nukleinsäure
      (C) STRANGFORM: Einzel
      (D) TOPOLOGIE: linear
   (ii) ART DES MOLEKÜLS: synthetisches Oligodesoxyribonukleotid
   (ix) MERKMALE:
      (A) NAME/SCHLÜSSEL: misc_feature
      (B) LAGE: 1..35
   (xi) SEQUENZBESCHREIBUNG: SEQ ID NO: 3:
   GGTACCTGGA TCCCGTTATA GCTCGGAATC CTCAA 35
(2) INFORMATION ZU SEQ ID NO: 4:
   (i) SEQUENZ CHARAKTERISTIKA:
      (A) LÄNGE: 25 Basenpaare
      (B) ART: Nukleinsäure
      (C) STRANGFORM: Einzel
      (D) TOPOLOGIE: linear
   (ii) ART DES MOLEKÜLS: synthetisches Oligodesoxyribonukleotid
   (ix) MERKMALE:
      (A) NAME/SCHLÜSSEL: misc_feature
      (B) LAGE: 1..25
   (xi) SEQUENZBESCHREIBUNG: SEQ ID NO: 4:
   TCAAGGGCAT CGGTCGACGC TCTCC 25
(2) INFORMATION ZU SEQ ID NO: 5:
   (i) SEQUENZ CHARAKTERISTIKA:
      (A) LÄNGE: 18 Basenpaare
      (B) ART: Nukleinsäure
      ( C) STRANGFORM: Einzel
      (D) TOPOLOGIE: linear
   (ii) ART DES MOLEKÜLS: synthetisches Oligodesoxyribonukleotid
   (ix) MERKMALE:
      (A) NAME/SCHLÜSSEL: misc_feature
      (B) LAGE: 1..18
   (xi) SEQUENZBESCHREIBUNG: SEQ ID NO: 5:
   GATCCTAAAT TGAATTGA 18
(2) INFORMATION ZU SEQ ID NO: 6:
   (i) SEQUENZ CHARAKTERISTIKA:
      (A) LÄNGE: 18 Basenpaare
      (B) ART: Nukleinsäure
      (C) STRANGFORM: Einzel
      (D) TOPOLOGIE: linear
   (ii) ART DES MOLEKÜLS: synthetisches Oligodesoxyribonukleotid
   (ix) MERKMALE:
      (A) NAME/SCHLÜSSEL: misc_feature
      (B) LAGE: 1..18
   (xi) SEQUENZBESCHREIBUNG: SEQ ID NO: 6:
   GATCTCAATT CAATTTAG 18
(2) INFORMATION ZU SEQ ID NO: 7:
   (i) SEQUENZ CHARAKTERISTIKA:
      (A) LÄNGE: 93 Basenpaare
      (B) ART: Nukleinsäure
      (C) STRANGFORM: Einzel
      (D) TOPOLOGIE: linear
   (ii) ART DES MOLEKÜLS: synthetisches Oligodesoxyribonukleotid
   (iii) ANTISENSE: NEIN
   (ix) MERKMALE:
      (A) NAME/SCHLÜSSEL: CDS
      (B) LAGE: 7..93
   (xi) SEQUENZBESCHREIBUNG: SEQ ID NO: 7:
   GATCTA GAA GCC GCC TTG GCC GAA GCC TTG GCC GAA GCC TTG GCC GAA       48
          Glu Ala Ala Leu Ala Glu Ala Leu Ala Glu Ala Leu Ala Glu
            1               5                  10
   CAC TTG GCC GAA GCC TTG GCC GAA GCC TTG GAA GCC TTG GCC GCC          93
   His Leu Ala Glu Ala Leu Ala Glu Ala Leu Glu Ala Leu Ala Ala
    15                  20                  25
(2) INFORMATION ZU SEQ ID NO: 8:
   (i) SEQUENZ CHARAKTERISTIKA:
      (A) LÄNGE: 29 Aminosäuren
      (B) ART: Aminosäure
      (D) TOPOLOGIE: linear
   (ii) ART DES MOLEKÜLS: Protein
   (xi) SEQUENZBESCHREIBUNG: SEQ ID NO: 8 :
   Glu Ala Ala Leu Ala Glu Ala Leu Ala Glu Ala Leu Ala Glu His Leu
     1               5                  10                  15
   Ala Glu Ala Leu Ala Glu Ala Leu Glu Ala Leu Ala Ala
               20                  25
(2) INFORMATION ZU SEQ ID NO: 9:
   (i) SEQUENZ CHARAKTERISTIKA:
      (A) LÄNGE: 93 Basenpaare
      (B) ART: Nukleinsäure
      (C) STRANGFORM: Einzel
      (D) TOPOLOGIE: linear
   (ii) ART DES MOLEKÜLS: synthetisches Oligodesoxyribonukleotid
   (iii) ANTISENSE: JA
   (ix) MERKMALE:
      (A) NAME/SCHLÜSSEL: misc_feature
      (B) LAGE: 1..93
   (xi) SEQUENZBESCHREIBUNG: SEQ ID NO: 9 :
   GATCTGGCGG CCAAGGCTTC CAAGGCTTCG GCCAAGGCTT CGGCCAAGTG TTCGGCCAAG 60
   GCTTCGGCCA AGGCTTCGGC CAAGGCGGCT TCT 93
(2) INFORMATION ZU SEQ ID NO: 10:
   (i) SEQUENZ CHARAKTERISTIKA:
      (A) LÄNGE: 106 Basenpaare
      (B) ART: Nukleinsäure
      (C) STRANGFORM: Einzel
      (D) TOPOLOGIE: linear
   (ii) ART DES MOLEKÜLS: synthetisches Oligodesoxyribonukleotid
   (iii) ANTISENSE: NEIN
   (ix) MERKMALE:
      (A) NAME/SCHLÜSSEL: CDS
      (B) LAGE: 7..105
   (xi) SEQUENZBESCHREIBUNG: SEQ ID NO: 10:
   GATCTA GGT TTG TTC GAA GCC ATT GAA GGT TTC ATT GAA AAC GGT TGG       48
          Gly Leu Phe Glu Ala Ile Glu Gly Phe Ile Glu Asn Gly Trp
           1               5                  10
   GAA GGT TTG GCC GAA GCC TTG GCC GAA GCC TTG GAA GCC TTG GCC GCC      96
   Glu Gly Leu Ala Glu Ala Leu Ala Glu Ala Leu Glu Ala Leu Ala Ala
    15                  20                  25                  30
   GGT GGT TCT A                                                       106
   Gly Gly Ser
(2) INFORMATION ZU SEQ ID NO: 11:
   (i) SEQUENZ CHARAKTERISTIKA:
      (A) LÄNGE: 33 Aminosäuren
      (B) ART: Aminosäure .
      (D) TOPOLOGIE: linear
   (ii) ART DES MOLEKÜLS: Protein
   (xi) SEQUENZBESCHREIBUNG: SEQ ID NO: 11:
   Gly Leu Phe Glu Ala Ile Glu Gly Phe Ile Glu Asn Gly Trp Glu Gly
   1 5 10 15
   Leu Ala Glu Ala Leu Ala Glu Ala Leu Glu Ala Leu Ala Ala Gly Gly
   20 25 30
   Ser
(2) INFORMATION ZU SEQ ID NO: 12:
   (i) SEQUENZ CHARAKTERISTIKA:
      (A) LÄNGE: 105 Basenpaare
      (B) ART: Nukleinsäure
      (C) STRANGFORM: Einzel
      (D) TOPOLOGIE: linear
   (ii) ART DES MDLEKÜLS: synthetisches Oligodesoxyribonukleotid
   (iii) ANTISENSE: JA
   (ix) MERKMALE:
      (A) NAME/SCHLÜSSEL: misc_feature
      (B) LAGE: 1..105
   (xi) SEQUENZBESCHREIBUNG: SEQ ID NO: 12:
   GATCTAGAAC CACCGGCGGC CAAGGCTTCC AAGGCTTCGG CCAAGGCTTC GGCCAAACCT 60
   TCCCAACCGT TTTCAATGAA ACCTTCAAGG CTTCGAACAA ACCTA 105
(2) INFORMATION ZU SEQ ID NO: 13:
   (i) SEQUENZ CHARAKTERISTIKA:
      (A) LÄNGE: 32 Basenpaare
      (B) ART: Nukleinsäure
      (C) STRANGFORM: Einzel
      (D) TOPOLOGIE: linear
   (ii) ART DES MOLEKÜLS: synthetisches Oligodesoxyribonukleotid
   (iii) ANTISENSE: NEIN
   (ix) MERKMALE:
      (A) NAME/SCHLÜSSEL: CDS
      (B) LAGE: 2..31
   (xi) SEQUENZBESCHREIBUNG: SEQ ID NO: 13:
   T ATG GCT AGC CAC CAT CAC CAT CAC CAT GGT G                          32
     Met Ala Ser His His His His His His Gly
       1               5                  10
(2) INFORMATION ZU SEQ ID NO: 14:
   (i) SEQUENZ CHARAKTERISTIKA:
      (A) LÄNGE: 10 Aminosäuren
      (B) ART: Aminosäure
      (D) TOPOLOGIE: linear
   (ii) ART DES MOLEKÜLS: Protein
   (xi) SEQUENZBESCHREIBUNG: SEQ ID NO: 14:
   Met Ala Ser His His His His His His Gly
     1               5                  10
(2) INFORMATION ZU SEQ ID NO: 15:
   (i) SEQUENZ CHARAKTERISTIKA:
      (A) LÄNGE: 34 Basenpaare
      (B) ART: Nukleinsäure
      (C) STRANGFORM: Einzel
      (D) TOPOLOGIE: linear
   (ii) ART DES MOLEKÜLS: synthetisches Oligodesoxyribonukleotid
   (iii) ANTISENSE: JA
   (ix) MERKMALE:
      (A) NAME/SCHLÜSSEL: misc_feature
      (B) LAGE: 1..34
   (xi) SEQUENZBESCHREIBUNG: SEQ ID NO: 15:
   GATCCACCAT GGTGATGGTG ATGGTGGCTA GCCA                                34